# EUROPEAN PATENT APPLICATION

(11) **EP 4 663 849 A1**
(43) Date of publication of application: **17.12.2025**
(21) Application number: 24753407.6
(22) Date of filing: 07.02.2024
(51) Int. Cl.: D21H 27/00, D21F 5/02

(54) **YANKEE DRYER MONITORING SYSTEM**

(30) Priority: 08.02.2023 JP 2023017927
(71) Applicant: Maintech Co., Ltd., Chiyoda-ku Tokyo 100-0005 (JP)
(72) Inventor: SEKIYA, Hiroshi, Tokyo 100-0005 (JP); IDE, Shota, Fuji-shi, Shizuoka 417-0003 (JP); WADA, Satoru, Fuji-shi, Shizuoka 417-0003 (JP); INAMATSU, Ryo, Fuji-shi, Shizuoka 417-0003 (JP); SAKATA Hitomaru, Fuji-shi, Shizuoka 417-0003 (JP); YUSA, Kazuyuki, Fuji-shi, Shizuoka 417-0003 (JP)
(74) Representative: Goddar, Heinz J.
(86) International application number: PCT/JP2024/004191
(87) International publication number: WO 2024/166964

(57) **Abstract**

To provide the monitoring system for the Yankee dryer that can know the appropriate timing to apply measure(s) to the Yankee dryer by monitoring the conditions of operation status of the Yankee dryer, without relying on empirical rules, and contain the lowering of the yield.

[Resolution] The present invention is the monitoring system of the Yankee dryer YD, which is comprised of the glossy metallic Yankee dryer YD; the chemical solution applying device N to apply the creping agent on the said Yankee dryer YD; a creping doctor CR that is placed on and pressed against the Yankee dryer YD; the light L to illuminate the Yankee dryer YD with light; the monitoring camera C to monitor the Yankee dryer YD; and the control device 10 that is connected to the monitoring camera C via a network, wherein the control device 10 is equipped with the acquisition mean 11 that acquires the image 20 captured by the monitoring camera C from the monitoring camera C; the calculation mean 12 that calculates average luminance that is an average value of luminance measured over every pixel in the pixels 21 in the image 20; the memory mean 13 that stores multiple average luminance over time obtained by repeating the acquisition mean 11 and calculation mean 12; and the judgement mean 14 that judges the state of the Yankee dryer to determine whether the average luminance exceeds a preset threshold.

## Description

### [Technical Field]

The present invention relates to the monitoring system of the Yankee dryer, and more specifically this invention relates to the monitoring system used to monitor conditions of the crepe process of the Yankee dryer in a paper machine.

### [Background Technology]

Paper production process is typically comprised of pulp preparation and papermaking processes. In general in the pulp preparation process, dried pulp sheet is defibrated, additives such as filler and sizing agent are added, stirred, and mixed to disperse the pulp. The papermaking process is comprised of wire, press, dry, and reel parts. In the wire part, pulp dispersion is placed on wire net to allow excessive water naturally falling out of the pulp dispersion. In the press part, the dispersed pulp (i.e., pulp dispersion) is fed into between a pair of press rolls and compressed against felt to absorb moisture in the pulp and to further dehydrate the pulp. In the dry part, the pulp that has gone through the press part makes contact on a heated Yankee dryer to further dry the paper. In the reel part, the dried paper is wound around a spool.

Furthermore, in such a papermaking process as above, the process called crepe process has been performed to form wrinkles on the paper when producing such household papers as tissue and toilet papers.

Incidentally, in the crepe process, paper dust remains on the Yankee dryer when the paper is peeled off from the Yankee dryer.

As a result, the paper dust is chronologically accumulated on the Yankee dryer, and this accumulation can be a factor that inhibits adhesiveness of the paper.

Although the creping agent used to enhance adhesiveness is applied on the surface of the Yankee dryer, the agent is gradually transferred onto the surface of the paper that is continuously guided to go through the paper machine; thus the amount of agent attached to the Yankee dryer decreases gradually.

This leads to a gradual lowering of adhesiveness of the paper.

In this way, if the crepe process continues to be used simply as it is, the adhesiveness of the paper on the Yankee dryer gradually decreases and the expected wrinkles will not be formed on the paper.

Therefore, several measures are currently being taken to prevent the adhesiveness from decreasing. These measures include the repeated applying of creping agent on the Yankee dryer (hereinafter, this is referred to as "the adhesiveness measure"); cleaning of the Yankee dryer to remove paper dust adhered on the Yankee dryer; replacement of creping doctor (hereinafter, this is referred to as "the paper dust measure"); etc.

However, these measures are taken at a timing decided based on empirical rules, and thus it is not necessarily possible to take measures at an appropriate timing in the case of, for example, change of operator(s).

If a measure is not taken at proper timing, it could result in a greatly lower yield of the crepe process.

To cope with the lowering of the yield, an attempt is being made to monitor the crepe process instead of the use of empirical rules.

For example, the following methods of monitoring a coating that is formed by the creping agent on the Yankee dryer are known.

That is, methods that monitor and arbitrarily control the formation of the coating of the agent containing performance enhancing agent (PEM: Polymer Electrolyte Membrane) on the surface of the creping cylinder are known (For example, see PTL 1). The following methods are included, (a) forming of the coating (film) on the creping cylinder (Yankee dryer) surface; (b) measurement of the thickness of the coating on the surface of the creping cylinder with the differential thickness measurement method that allows the measurement of multiple equipment/devices without direct contact with the coating; (c) arbitrary adjustment of the coating formation on one or more defined zones of the creping cylinder taking the coating thickness into account such that the coating thickness on the creping cylinder surface can be uniform; and (d) arbitrary control of other factor(s) of the coating than the coating thickness on the creping cylinder by making arbitrary use of additional device(s).

By relying on these methods of monitoring the formation of the coating, the amount of the creping agent applied on the Yankee dryer can be monitored, and thus the timing to apply the agent can be known without relying on empirical rules.

### [Citation List]

### [Patent Literature]

[PTL 1] Patent 2012-505322

### [Summary of Invention]

### [Technical Problem]

However, it is not sufficient only to monitor the formation of the coating with the method described in the above PTL 1, where the method of monitoring the coating formation is discussed.

That is, in the crepe process, even if the coating is perfectly formed on the Yankee dryer, it is not possible to prevent paper dust from accumulating on the Yankee dryer and to prevent the yield from lowering due to this accumulation, because paper dust always adheres to Yankee dryers.

The present invention has been devised in view of the above circumstances, and objective thereof is to provide the Yankee dryer with a monitoring system that is capable of knowing the appropriate timing to apply the measure(s) on the Yankee dryer by monitoring the state of the Yankee dryer without relying on empirical rules, thereby suppressing a decrease in the yield.

### [Means for Solving the Problems]

The inventors of the present invention considered that the Yankee dryer, to which the creping agent and paper dust adhere, could be directly monitored.

The inventors focused on the luminance of the Yankee dryer when the Yankee dryer is illuminated with light; found that the above problem could be solved by providing a mean for calculating an average luminance and a mean for determining whether the said average luminance exceeds a prescribed threshold; and thereby the inventors completed the present invention.

The present invention is the Yankee dryer monitoring system used to monitor the state of the Yankee dryer during the crepe process, and the system is comprised of a glossy metal Yankee dryer; a chemical solution applying device for spraying the creping agent on the said Yankee dryer; a creping doctor that is placed on and pressed against the Yankee dryer; a light that irradiates the Yankee dryer; a monitoring camera that monitors the Yankee dryer; a control device that is connected to the monitoring camera via a network; as well as a control device consisting of an acquisition mean that acquires an image of an arbitrary area on the surface of the operating Yankee dryer, where the area is captured by the monitoring camera; a calculation mean that calculates average luminance which is an average value of luminance measured respectively for each pixel of the image; a memory mean that chronologically memorizes multiple luminance averages obtained with repetition of acquisition and a calculation mean; and a judgement mean that judges the state of the Yankee dryer based on whether the average luminance exceeds a preset threshold.

In the Yankee dryer monitoring system of the present invention, when a widthwise direction of the Yankee dryer in the image is defined to be an X axis, and a direction orthogonal to the said X axis is defined to be a Y axis, it is preferable that the calculation mean can calculate an average luminance in pixel rows that are continuously running in the Y axis direction, and that the calculation mean can also calculate the average luminance in pixel rows that are continuously running in the X axis direction.

In the Yankee dryer monitoring system of the present invention, it is preferable that the monitoring camera is a line scan camera.

In the Yankee dryer monitoring system of the present invention, it is preferable that the light and monitoring camera are directed to an area of the Yankee dryer that is in contact with the paper.

### [Advantageous Effects of Invention]

In the Yankee dryer monitoring system of the present invention, the control device has the calculation mean for calculating the average luminance of images in an arbitrary area of the Yankee dryer illuminated by the light and it has the memory mean for chronologically storing the said average luminance, so that chronological change of the average luminance of the surface of the Yankee dryer in the crepe process can be known.

That is, when the creping agent and paper dust (hereinafter, these are collectively referred to as "attached materials") are attached on the Yankee dryer, average luminance of the image decreases in proportion to the total amount of the attached materials. Therefore, it becomes possible to chronologically know the change of the amount of the attached materials.

Note that, since the Yankee dryer is made of glossy metal, the Yankee dryer has a high luminance (i.e., it is bright), and thus it makes changes in the average luminance more easily detectable by the monitoring system of the present invention.

In addition, since the control device has the judgement mean for determining whether the average luminance exceeds the prescribed threshold, this judgement mean can tell an appropriate timing to take the adhesiveness measure of applying the creping agent on the Yankee dryer, to take the paper dust measure that cleans the Yankee dryer by removing paper dust attached on the Yankee dryer and/or by replacing the creping doctor, or to take any other measure(s).

Note that the crepe process can be continued as long as the average luminance does not exceed its threshold.

Thus, with the monitoring systemin of the Yankee dryer system, it is possible to know the appropriate timing for taking the measure(s) such as the adhesiveness measure and paper dust measure on the Yankee dryer without resorting to the empirical rules and, as a result, it becomes possible to prevent yield from lowering in advance.

In the Yankee dryer monitoring system of the present invention, it becomes possible to know the position where the average luminance in the X-axis direction exceeds the threshold because the calculation mean further calculates the average luminance in every pixel row in the X-axis direction.

In the Yankee dryer monitoring system of the present invention, its calculation mean can classify the luminance of the Yankee dryer into three luminance levels based on the numerical feature of the luminance of each pixel. They are the first luminance of an area where the paper dust adhered on the Yankee dryer, the second luminance of an area where the creping agent adhered on the Yankee dryer, and the third luminance of an area where the paper dust as well as creping agent adhered on the Yankee dryer.

Then, just as described above, it becomes possible, from these averages of luminance, to monitor the surface state of the Yankee dryer in more in detail by determining whether the average luminance exceeds the threshold.

As a result, the above-described adhesiveness measure, paper dust measure, and the like can be implemented more effectively.

When the monitoring camera in the Yankee dryer monitoring system of the present invention is a line scan camera, there are advantages that images can have higher resolution than those taken by so called area scan camera, and that images are less likely to have uneven luminance due to the lighting because of the narrow imaging area.

In the Yankee dryer monitoring system of the present invention, it becomes possible to install the light and monitoring camera in a sufficient space by directing the light and monitoring camera to the area of the Yankee dryer that is in contact with the paper.

Incidentally, since the Yankee dryer is used to dry paper that is conveyed at a high speed, a large area of the outer periphery of the Yankee dryer is in contact with the paper whereas, in general, the area that is not in contact with the paper is limited.

### [Brief Description of Drawings]

[Fig. 1] Fig. 1 is the schematic drawing of the side view of the dry part and reel part that are included in an embodiment of the monitoring system of the Yankee dryer according to the present invention.
[Fig. 2] Fig. 2 is a block diagram explaining the control device of the monitoring system of the Yankee dryer according to the present embodiment.
[Fig. 3] Fig. 3 is a flowchart showing the operation of the monitoring system of the Yankee dryer according to the present embodiment.
[Fig. 4] Fig. 4 is an enlarged diagram of a part of the image to explain pixels used in the monitoring system of the Yankee dryer according to the present embodiment.

### [Description of Embodiments]

Hereinafter, a preferred embodiment of the present invention is described in detail, referring to drawing(s) as necessary.

Note that, in these drawings, the same reference number is allocated to the same elements, and redundant description is omitted.

Note that, unless otherwise specified, the positional relationship such as the top, bottom, left, right, etc. is based on the positional relationship shown in the drawing(s).

Furthermore, note that the dimensional ratios (i.e., aspect ratios) shown in the drawings are not limited to the ratios shown in the drawings.

The monitoring system of the Yankee dryer according to the present invention is a system for monitoring the state of the Yankee dryer while so-called crepe process is being performed in the dry part of the papermaking process.

In the present invention, the papermaking process at least includes the wire part, press part, dry part, and reel part.

In addition, the crepe process is performed by applying the creping agent on the Yankee dryer in the dry part of the papermaking process to attach the paper on the Yankee dryer and forcibly to peel off the paper attached on and pressed against the Yankee dryer from the creping doctor that is in contact with the Yankee dryer; and, at the same time, to make the rotation speed of the paper take-up roller (spool) of the paper, on which the crepe process has already been applied, slower than the rotation speed of the feed roller, which feeds the paper to the dry part.

Note that the paper on which the crepe process is applied is used as household paper, and, more specifically, it is used as tissue paper, toilet paper, paper towel, kitchen paper, paper napkin, paper diaper, artificial flower, etc. depending on the material of the paper.

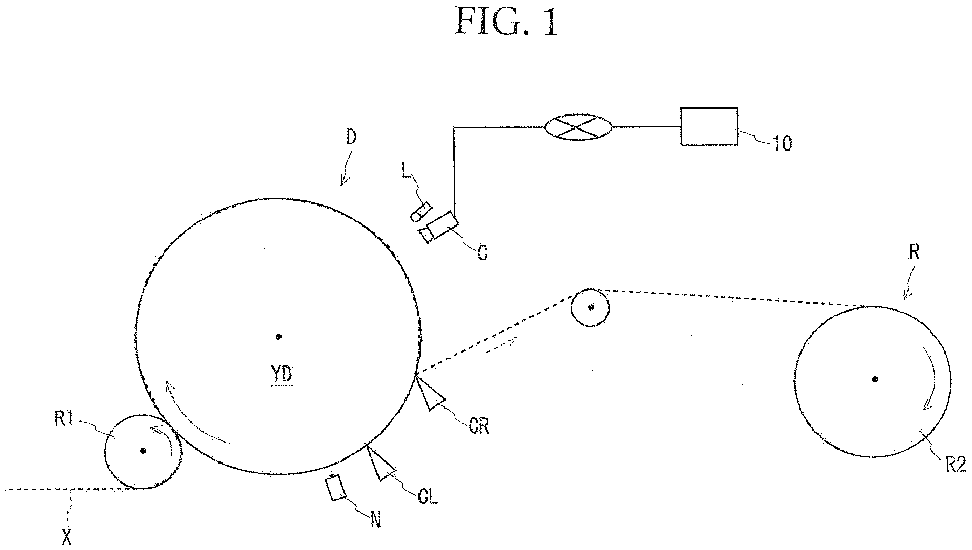

Fig. 1 is a schematic side view of the dry part and reel part included in one embodiment of the monitoring system of the Yankee dryer according to this invention.

As shown in Fig. 1, in the dry part D, the paper X fed from the feed roller R1 is dried while in contact with the surfaces of the Yankee dryer YD.

Then, the crepe process is applied on the paper X by forcibly peeling off the paper X with the creping doctor CR.

In the reel part R, the paper X, on which the crepe process has been applied, is wound up by the spool R2.

The monitoring system of the Yankee dryer according to the present embodiment is used in the dry part D of the papermaking process.

That is, the monitoring system of the Yankee dryer includes the Yankee dryer YD; chemical solution applying device N to apply the creping agent on the said Yankee dryer YD; creping doctor CR and cleaning doctor CL that are placed on and pressed against the Yankee dryer YD; light L to illuminate light on the Yankee dryer YD; monitoring camera C to monitor the Yankee dryer YD; and control device 10 connected to the monitoring camera C via a network.

In the monitoring system of the Yankee dryer YD, the monitoring camera C captures an image of an arbitrary area of the Yankee dryer YD from above the paper X where the Yankee dryer YD is illuminated with light from the light L; the control device 10 calculates the average luminance of the image and stores the chronological change of the said average luminance; and thus it is possible to know the chronological change in the average luminance of the surface of the Yankee dryer YD during the crepe process.

In addition, the control device 10 judges whether the average luminance exceeds the prescribed threshold, and when the average luminance exceeds the prescribed threshold, it becomes possible to know the appropriate timing for taking such paper dust measure(s) as applying the creping agent on the Yankee dryer YD (i.e., adhesiveness measure), cleaning of paper dust to remove the dust adhered on the Yankee dryer YD, or replacing the doctor.

Note that details of the above statement are elaborated later.

Thus, the monitoring system of the Yankee dryer YD can know the appropriate timing for taking measure(s) against the Yankee dryer YD without resorting to empirical rules, and, as a result, it becomes possible to suppress a decrease in yield in advance.

Hereinafter, each system, device, etc., configuring the present invention is further explained in more detail.

The Yankee dryer YD is a so-called rotary heating drum, which can guide the paper, and, at the same time, can heat, and dry the paper by causing the paper to make contact with the surface of the Yankee dryer YD.

The Yankee dryer YD is made of cast iron (metal) and has a glossy surface.

Therefore, the Yankee dryer YD has an advantage that the luminance is increased (the Yankee dryer YD becomes brighter) when it is illuminated with the light, and the change of the average luminance is more easily recognizable.

The Yankee dryer YD can adjust the temperature of its surface.

Therefore, as described later, when the judgement mean judges that the overall average luminance is lower than the threshold, for example, there is a risk that the paper dust may be scorched on the Yankee dryer YD, and thus it is possible to take the measure to lower the temperature of the surface of the Yankee dryer YD.

The chemical solution applying device N is a device for applying the creping agent on the Yankee dryer YD.

Specifically, the chemical solution applying device N can apply the creping agent across the entire width of the Yankee dryer YD while sliding back and forth the device on a rail (not shown) that extends the width of the Yankee dryer YD.

Therefore, as described later, when the judgement mean judges that the overall average luminance is higher than the threshold, it is possible to, for example, repeat the reciprocating sliding motion of the chemical solution applying device N in order to repeatedly apply the creping agent on the Yankee dryer YD.

In addition, the chemical solution applying device N can also locally apply the creping agent on the Yankee dryer YD by controlling the chemical solution discharge timing of the chemical solution applying device N.

Therefore, for example, when the judgement mean judges that the average luminance of pixel row at the specific position is higher than the threshold with the judgement mean described later, an action to apply the creping agent only to this specific position can be taken.

Note that, the creping agent is generally applied in diluted or dispersed state with such solvent as water.

For such creping agent as above, a commonly known creping agent can be appropriately adopted.

For example, lubricants composed of an organic solid and/or inorganic solid lubricant; a dispersant for dispersing the lubricant; thermosetting polymer for fixing on the lubricant on the surface of the Yankee dryer YD; and creping agent composition that includes water as a solvent which are cited in PCT International Publication No. WO 2010/044280 can be used as the creping agent. To be more specific, the active component (nonvolatile component) thereof can be adopted as the creping agent.

The creping doctor CR is a doctor having a doctor blade (edge) used to perform the crepe process, and a commonly known doctor can be appropriately adopted.

The creping doctor CR is placed on and pressed against the Yankee dryer YD at a prescribed angle and prescribed pressing pressure.

Note that such above angle and pressing pressure can be set arbitrarily according to the desired shape of crepes.

In general, as the crepe process is continued, the doctor blade of the creping doctor CR is abraded.

When the blade is abraded, the paper cannot be sufficiently peeled off, and thus the amount of the paper dust adhering to the Yankee dryer YD increases.

Therefore, as described later, when the cycle in which judgement mean judge that the overall average luminance was lower than the threshold becomes shorter, for example, it can be understood that it is time to replace the creping doctor CR.

The cleaning doctor CL is placed on and pressed against the Yankee dryer YD downstream of the creping doctor CR and in the same rotational direction as that of the Yankee dryer YD.

The cleaning doctor CL is for scraping off paper dust adhered to the Yankee dryer YD, and as with the creping doctor CR, the blade of the cleaning doctor abrades when the crepe process is continued.

Therefore, as described later, when the cycle in which judgement mean judges that the overall average luminance is lower than the threshold becomes shorter, for example, it can be understood as the timing to replace the cleaning doctor CL.

The monitoring camera C captures an image of an arbitrary area on the surface of the Yankee dryer YD from above the paper X while the Yankee dryer YD is running.

As such monitoring camera C, a video camera, a line scan camera, an area scan camera, etc. can be adopted.

Among these cameras, the line scan camera is preferably used as the monitoring camera C.

In this case where the line scan camera is used, the resolution of the image can be higher than that of the image taken by the area scan camera, and, since the imaging area is small, there is an advantage that unevenness due to lighting is less likely to occur.

When the monitoring camera C is a line scan camera, a still image having a vertical length of one pixel and an arbitrary horizontal width is captured, and the acquisition mean 11 that acquires the still images and combines the images in the vertical direction, thereby obtaining the entire image of a prescribed area.

At this time, the imaging speed of the line scan camera is preferably 25,000 times/second or more, and it is more preferable to take an image at 50,000 times/second or more taking the conveying speed of the paper sheet X (500 to 2,000m/second) into account.

It is also preferable that the monitoring camera C captures the paper X as a monochrome image.

This reduces the load of image capacity and enables the calculation mean 12 to easily calculate the luminance of each pixel.

The light L illuminates the area that has to be shot by the monitoring camera C.

For such light L, a commonly known light can be appropriately adopted.

Note that, as the light L, a specular reflection type LED lighting is preferably used.

The monitoring camera C and light L are directed to an area of the Yankee dryer YD where it is in contact with the paper.

Incidentally, in the monitoring system of the Yankee dryer YD, the change in the average luminance can be sufficiently known even if the Yankee dryer YD is pictured through the paper X. This is because the paper X is sufficiently thin.

In this case, there is an advantage that there is more space to install the monitoring camera C and light L can be installed.

As a result, the equipment and/or device used in the monitoring system of the Yankee dryer YD could be installed on an existing paper machine even after the machine has already been installed.

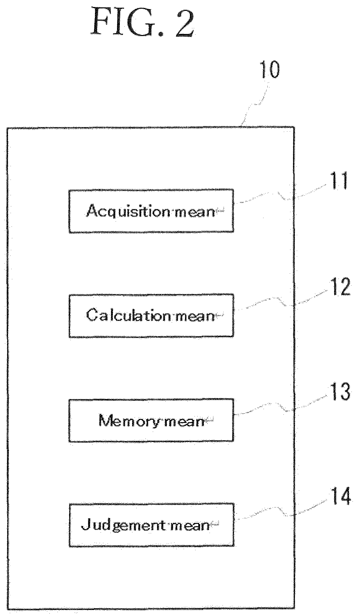

Fig. 2 is a block diagram for explaining a control device of the monitoring system of the Yankee dryer according to the present embodiment.

As shown in Fig. 2, the control device 10 is at least equipped with the acquisition mean 11, calculation mean 12, memory mean 13, and judgement mean 14.

Note that the control device 10 is a computer in which prescribed programs have been installed.

In the monitoring system of the Yankee dryer YD, various processes are sequentially executed in the control device 10 after the images taken by the monitoring camera C have been acquired.

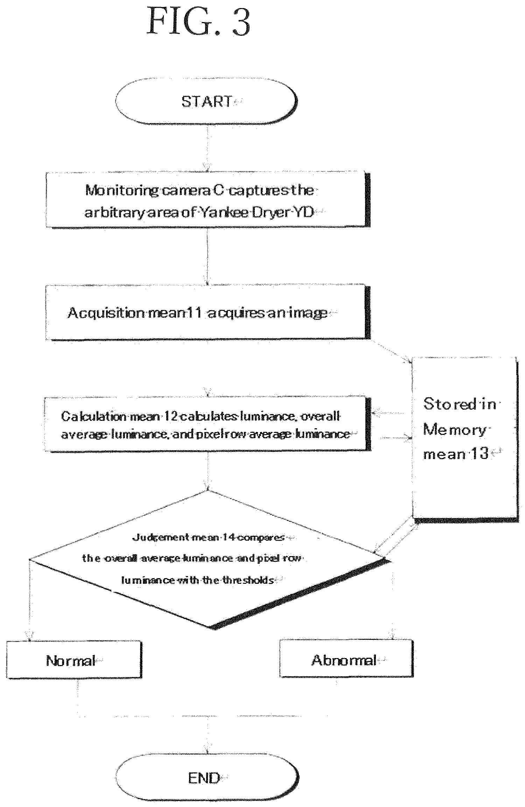

Fig. 3 is a flowchart that shows operations of the monitoring system of the Yankee dryer according to the present embodiment.

The acquisition mean 11 is connected to the monitoring camera C via a wired or wireless network, and transmits command(s) to the monitoring camera C in order to take images of an arbitrary area of the surface of the Yankee dryer YD while it is operating (from above the paper X).

Then, as shown in Fig. 3, the monitoring camera C captures an image of the area.

After images have been taken by the monitoring camera C, the acquisition mean 11 acquires these images.

The acquired images are stored in the memory mean 13.

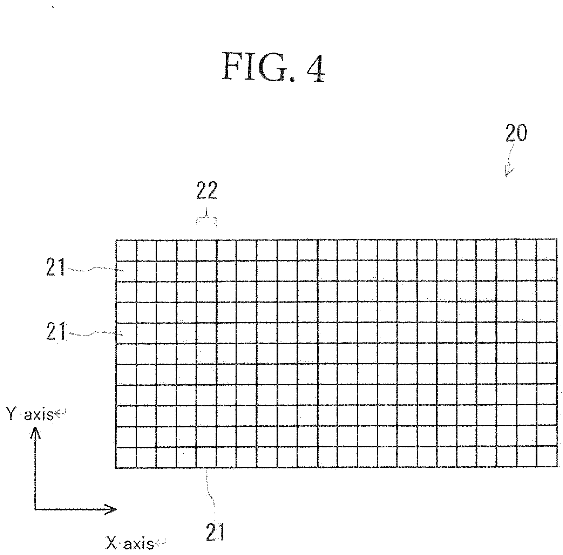

Fig. 4 is a diagram of an enlarged part of an image of the monitoring system of the Yankee dryer according to the present embodiment, and it is provided to explain pixels used in the monitoring system.

As illustrated in Fig. 4, pixels 21 that form the image 20 are arranged in a lattice pattern.

The calculation mean 12 retrieves the image 20 stored in the memory mean 13 and calculates the luminance of every pixel in the pixels 21 for the said entire image 20.

The number of pixels in the above image is preferably 2,000 pixels or more in the widthwise direction from the viewpoint of improving accuracy.

The calculation mean 12 then calculates an average luminance which is an average value of the overall luminance (hereinafter, also referred to as "overall average luminance" for convenience), by using the luminance of each one of the obtained pixels.

Note that the calculated overall average luminance is stored in the memory mean 13.

Here, as shown in Fig. 4, when the widthwise direction of the Yankee dryer in the image 20 is defined as an X axis and a direction orthogonal to this X axis is defined as a Y axis, a row of pixel 21 in the X-axis direction and multiple pixels of pixel 21 connected from the said pixel 21 in the Y-axis direction are referred to as a pixel row 22.

That is, the image 20 is formed by juxtaposing the multiple pixel row 22 in parallel to the X-axis direction.

On the other hand, the calculation mean 12 calculates an average luminance in a pixel row (hereinafter, also referred to as "pixel row average luminance" for convenience) by making use of the luminance of each obtained pixel in every pixel row.

Note that the calculated average luminance in the pixel row is stored in the memory mean 13.

Referring to Fig. 3, the memory mean 13 at least stores the images acquired by the acquisition mean 11; luminance of every pixel in the said image calculated by the calculation mean 12; overall average luminance and average luminance of the pixel row of the image calculated by the calculation mean 12; and judgement result obtained by the judgement mean 14.

The data obtained by repeating the acquisition mean 11 and calculation mean 12 is chronologically memorized.

Note that this data can be retrieved in a timely manner.

The judgement mean 14 judges the state of the Yankee dryer depending on whether the overall average luminance and pixel row average luminance stored in the memory mean 13 exceed respective preset thresholds.

That is, when the overall average luminance and pixel row average luminance do not exceed the threshold, the Yankee dryer is judged as "normal", and when they exceed the threshold, the Yankee dryer is judged as "abnormal".

Note that the judged result is stored in the memory mean 13.

Note that the threshold can be set arbitrarily.

For example, after the normal crepe process, the threshold should be set based on the correlation between the timing of conducting the measure(s), chronological changes in the overall average luminance, and pixel row average luminance obtained from the process.

Note that if it is necessary to know the timing to take the measure(s) in advance, it is only necessary to narrow the range of the threshold.

When the overall average luminance and pixel row average luminance are judged to be "normal" by the judgement mean 14, the crepe process is continued.

On the other hand, when the overall average luminance is judged to be "abnormal", the above-described measure(s) is taken.

This makes it possible to know the timing to take the measure(s).

As a result, it becomes possible to prevent the yield from decreasing in advance. When the pixel row average luminance is judged to be "abnormal", the above-described measure is taken locally.

This makes it possible, from the position of the pixel row judged as "abnormal", to know a position where the average luminance in the X-axis direction exceeds the threshold and to know the timing to take measure(s) for that position.

As a result, it becomes possible to prevent the yield from decreasing in advance.

For the measure taken, for example, when the overall average luminance exceeds the upper limit of the threshold, it can be said that the amount of the creping agent and paper dust in the Yankee dryer YD is small making the paper X more adherent.

That is, the above described adhesiveness measure that applies the creping agent repeatedly is taken. When the overall average luminance is less than the lower limit of the threshold, it can be said that too much of the creping agent and paper dust are adhered to the Yankee dryer YD, and therefore, the paper dust measure must be performed to remove the paper dust adhering to the Yankee dryer YD.

In addition, to suppress the adhered paper dust from being burnt on the Yankee dryer YD, the temperature of the surface of the Yankee dryer YD is lowered as necessary.

When the average luminance of the pixel rows exceeds the upper or lower limit of the threshold, the measure(s) similar to those described above is taken at the place where the average luminance of the pixel is higher than the upper threshold or lower than the lower threshold.

In addition, when the cycle in which the said overall average luminance was shorter than the lower threshold becomes shorter while observing the chronological change of the overall average luminance, it is considered that the paper X is not sufficiently peeled off or that the paper dust is not sufficiently scraped off. Therefore, it can be understood as the timing to replace the creping doctor CR in the case of the former, or to replace the cleaning doctor CL in the case of the latter.

Note that which factor (i.e., the paper X is not fully peeled off or the paper dust is not fully scraped out) is making the cycle shorter can be visually distinguishable.

In a case where it is judged that the overall average luminance exceeds the lower limit of the threshold and it is not improved even after the above-described measure(s) is taken, it can be understood that it is time to clean or replace the monitoring camera C or light L.

Although the preferred embodiment of the present invention has been described as above, the present invention is not limited to the above embodiment.

The monitoring system of the Yankee dryer YD according to the present embodiment includes the cleaning doctor CL, but it is not essential.

In the monitoring system of the Yankee dryer YD according to the present embodiment, the chemical solution applying device N that applies the creping agent to the entire width of the Yankee dryer YD while sliding back and forth along a rail that is extending in the widthwise direction of the Yankee dryer YD is adopted, but, instead of the type that slides back and forth to apply the creping agent, the chemical applying device N can be a device (i.e., shower type device) that showers the creping agent from multiple nozzles located on the main body portion of the Yankee dryer YD where the nozzles extend the width of the Yankee dryer YD.

In the monitoring system of the Yankee dryer YD according to the present embodiment, the monitoring camera C and light L are directed to an area of the Yankee dryer YD that is in contact with the paper X, but the direction of the camera is not essential.

That is, although the installation position of the monitoring camera C and light L is limited, the monitoring camera C and light L can be directed to a portion of the Yankee dryer YD which is not in contact with the paper X as long as there is sufficient space available.

In the monitoring system of the Yankee dryer YD according to the present embodiment, the control device 10 is equipped with the acquisition mean 11; calculation mean 12; memory mean 13; and judgement mean 14, but it can obviously be equipped with such display devices as a monitor, such input device as mouse, and the like.

In the monitoring system for the Yankee dryer YD according to the present embodiment, the control device has a learning device, and the said learning device can learn the judgement result made by the judgement mean, so that the timing to take the measure can be changed based on what has been learned.

### [Industrial Applicability]

The monitoring system of the Yankee dryer of the present invention can be used as a system for monitoring the Yankee dryer during the crepe process in the papermaking process.

Relying on the Yankee dryer monitoring system of the present invention, it is possible by monitoring the state of the Yankee dryer to know the appropriate timing for taking measure(s) for the Yankee dryer without relying on empirical rules, and as a result, it becomes possible to suppress a decrease in yield.

### [Explanation of References]

- 10: Control device
- 11: Acquisition mean
- 12: Calculation mean
- 13: Memory mean
- 14: Judgement mean
- 20: Image
- 21: Pixel
- 22: Pixel row
- C: Monitoring camera
- CR: Creping doctor
- CL: Cleaning doctor
- D: Dry part
- L: Light
- N: Chemical solution applying device
- R: Reel part
- R1: Feed roller
- R2: Spool
- X: Paper
- YD: Yankee Dryer

## Claims

1. A monitoring system in the Yankee dryer for monitoring a state of the Yankee dryer in the crepe process, which is comprised of:
a glossy metal Yankee dryer; a chemical solution applying device for applying a creping agent on the said Yankee dryer; the preceding creping doctor that is placed on and pressed against the Yankee dryer; a light for illuminating the preceding Yankee dryer; a monitoring camera for monitoring the preceding Yankee dryer; and a control device connected to the said monitoring camera via a network.
The preceding control device comprises of the monitoring system of the Yankee dryer that is provided with:
an acquisition mean configured to acquire, from the preceding monitoring camera, an image of an arbitrary area of the surface of the preceding Yankee dryer in operation, the image being captured by the preceding monitoring camera;
a calculation mean configured to calculate an average luminance that is an average value of luminance measured for respective pixels of the preceding image;
a memory mean configured to store multiple of average luminance obtained by repeating the preceding acquisition mean and preceding calculation mean over time; and
a judgement mean that judges the state of the preceding Yankee dryer to determine whether or not the preceding average luminance exceeds a preset threshold.

2. The Yankee dryer monitoring system according to Claim 1, wherein when the widthwise direction of the Yankee dryer in the image is the X axis and the direction orthogonal to the X axis is the Y axis, the calculation means further calculates the average luminance in a series of pixel rows in the Y axis direction for each pixel row in the X axis direction.

3. The monitoring system according to Claim 1, wherein the preceding monitoring camera is a line scan camera.

4. The monitoring system according to Claim 1, wherein the preceding light and preceding monitoring camera are directed toward an area of the preceding Yankee dryer that is in contact with the paper.
